# EUROPEAN PATENT APPLICATION

(11) **EP 4 129 152 A1**
(43) Date of publication of application: **08.02.2023**
(21) Application number: 21780377.4
(22) Date of filing: 02.04.2021
(51) Int. Cl.: A61B 1/045

(54) **MEDICAL IMAGE PROCESSING APPARATUS, ENDOSCOPE SYSTEM, OPERATION METHOD FOR MEDICAL IMAGE PROCESSING APPARATUS, AND PROGRAM FOR MEDICAL IMAGE PROCESSING APPARATUS**

(30) Priority: 03.04.2020 JP 2020067362
(71) Applicant: FUJIFILM Corporation, Tokyo 106-8620 (JP)
(72) Inventor: ENDO, Maiko, Tokyo 106-8620 (JP)
(74) Representative: Jeffrey, Philip Michael
(86) International application number: PCT/JP2021/014299
(87) International publication number: WO 2021/201272

(57) **Abstract**

There are provided a medical image processing apparatus, an endoscope system, a method of operating a medical image processing apparatus, and a program for a medical image processing apparatus capable of allowing a user to recognize a decrease in visibility of highlight display.

A processor device (16) includes an image signal acquisition unit (50), a display control unit (58), and a region-of-interest detection mode image processing unit (64). The image signal acquisition unit (50) acquires an image signal from an endoscope (12). The region-of-interest detection mode image processing unit (64) detects a region-of-interest from the endoscopic image. The display control unit (58) superimposes a highlight display of the region-of-interest on the endoscopic image and displays the endoscopic image on which the highlight display is superimposed. The processor device determines a visibility of the highlight display from image information of the endoscopic image and the highlight display, and notifies a user of a determination result.

## Description

### BACKGROUND OF THE INVENTION

### 1. Field of the Invention

The present invention relates to a medical image processing apparatus, an endoscope system, a method of operating a medical image processing apparatus, and a program for a medical image processing apparatus capable of detecting a region-of-interest such as a lesion portion.

### 2. Description of the Related Art

In a medical field, image diagnosis such as diagnosis of a disease of a patient and follow-up are performed by using medical images such as endoscopic images, X-ray images, computed tomography (CT) images, and magnetic resonanse (MR) images. Based on such image diagnosis, a doctor or the like make a decision on a treatment policy.

In recent years, in the image diagnosis using medical images, the medical images are analyzed, and regions-of-interest that should be carefully observed such as lesions and tumors in organs are automatically detected. In particular, by performing machine learning such as deep learning, accuracy in detection of the regions-of-interest is dramatically improved.

WO2018/198161A and WO2017/081976A disclose a medical image processing apparatus that performs image processing based on detection information in a case where a region-of-interest such as a lesion portion is detected from a medical image. The medical image processing apparatus disclosed in WO2018/198161A and WO2017/081976A performs highlight processing of superimposing a highlight display for highlighting the region-of-interest on the medical image.

### SUMMARY OF THE INVENTION

However, in the medical image processing apparatus disclosed in WO2018/198161A and WO2017/081976A, a visibility of the highlight display is not considered. As a result, depending on a color of a subject in the medical image, the presence or absence of an object existing in the subject, and the like, the highlight display may be assimilated with surroundings or may be less conspicuous with respect to surrounding portions. In a case where the visibility of the highlight display is decreased in this way, a doctor may not notice the region-of-interest.

An object of the present invention is to provide a medical image processing apparatus, an endoscope system, a method of operating a medical image processing apparatus, and a program for a medical image processing apparatus capable of allowing a user to recognize a decrease in visibility of the highlight display.

According to an aspect of the present invention, there is provided a medical image processing apparatus including a processor, in which the processor is configured to acquire a medical image, detect a region-of-interest in the medical image, set a highlight display for highlighting the detected region-of-interest and superimpose and display the highlight display on the medical image, determine a visibility of the highlight display from image information acquired from the medical image in which the region-of-interest is detected and the highlight display which is set, and notify a user of a determination result of the visibility.

Preferably, the processor is configured to acquire the image information from an inside of the highlight display in the medical image. Alternatively, preferably, the processor is configured to acquire the image information from an outside of the highlight display in the medical image.

Preferably, the processor is configured to acquire a color difference between the medical image and the highlight display from color information calculated from the image information and color information calculated from the highlight display, and determine the visibility from the color difference. Preferably, the processor is configured to calculate, as the color information, an average value calculated from the image information.

Preferably, the processor is configured to display, as the highlight display, a frame-shaped figure surrounding the region-of-interest, and determine the visibility from a thickness of a line of the frame-shaped figure with respect to the region-of-interest. Further, preferably, the processor is configured to display, as the highlight display, a frame-shaped figure surrounding the region-of-interest, and determine the visibility from a similarity of the frame-shaped figure to the region-of-interest.

Preferably, the processor is configured to display the determination result on a display screen. In addition, preferably, the processor is configured to calculate a numerical index value as the determination result of the visibility, and display the determination result on a display screen. Further, preferably, the processor is configured to display the index value as the notification in a case where the index value is equal to or smaller than a preset threshold value.

Preferably, the processor is configured to use, as the index value, a color difference calculated from the image information and the highlight display. Further, the processor may be configured to calculate a numerical index value from the determination result of the visibility, and display identification information or an identification figure according to the index value.

Preferably, the processor is configured to determine the visibility based on the presence or absence of an object other than a detection target that exists in an inside of the highlight display. Preferably, the processor is configured to determine that the object other than the detection target exists in a case where an area ratio of a portion at which brightness or luminance of the inside of the highlight display is equal to or higher than a second threshold value to a range in the inside of the highlight display in the medical image is equal to or higher than a third threshold value.

The processor may be configured to display the determination result on the display screen different from a display screen on which the medical image is displayed. Preferably, the processor is configured to automatically store the medical image in which the region-of-interest is detected in a case where the index value is equal to or smaller than a preset first threshold value. Further, preferably, the processor is configured to perform warning for a user in a case where the index value is equal to or smaller than a preset first threshold value.

According to another aspect of the present invention, there is provided an endoscope system including a light source device, an endoscope, a processor, and a display unit. The processor is configured to acquire a medical image, detect a region-of-interest in the medical image, set a highlight display for highlighting the detected region-of-interest and superimpose the highlight display on the medical image and display the medical image on which the highlight display is superimposed on the monitor, determine a visibility of the highlight display from image information acquired from the medical image in which the region-of-interest is detected and the highlight display which is set, and notify a user of a determination result of the visibility. The light source device emits an illumination light beam for illuminating an observation target. The endoscope includes an imaging sensor which images the observation target illuminated with the illumination light beam. The monitor displays a medical image obtained by performing signal processing on an image signal which is output by the imaging sensor.

According to still another aspect of the present invention, there is provided a method of operating a medical image processing apparatus, the method including: a step of acquiring a medical image; a step of detecting a region-of-interest in the acquired medical image; a step of setting a highlight display for highlighting the detected region-of-interest and superimposing and displaying the highlight display on the medical image; a step of determining a visibility of the highlight display from image information acquired from the medical image in which the region-of-interest is detected and the highlight display which is set; and a step of notifying a user of a determination result of the visibility.

According to still another aspect of the present invention, there is provided a program for a medical image processing apparatus, the program being installed in the medical image processing apparatus that acquires a medical image and performs image processing on the medical image, the program causing a computer to realize: a function of acquiring the medical image; a function of detecting a region-of-interest in the medical image; a function of setting a highlight display for highlighting the detected region-of-interest and superimposing and displaying the highlight display on the medical image; a function of determining a visibility of the highlight display from image information acquired from the medical image in which the region-of-interest is detected and the highlight display which is set; and a function of notifying a user of a determination result of the visibility.

According to the present invention, the user can recognize a decrease in visibility of the highlight display.

### BRIEF DESCRIPTION OF THE DRAWINGS

Fig. 1 is an external view of an endoscope system.
Fig. 2 is a block diagram illustrating a function of the endoscope system including a plurality of LED light sources according to a first embodiment.
Fig. 3 is a graph illustrating spectral spectra of a violet light beam V, a blue light beam B, a blue light beam Bx, a green light beam G, and a red light beam R.
Fig. 4 is a graph illustrating a spectral spectrum of a normal light beam according to the first embodiment.
Fig. 5 is a graph illustrating a spectral spectrum of a special light beam according to the first embodiment.
Fig. 6 is a block diagram illustrating functions of a region-of-interest detection mode image processing unit and a display control unit.
Fig. 7 is an explanatory diagram illustrating a highlight region which is set in a case where the display control unit performs highlight display of a region-of-interest.
Fig. 8 is an explanatory diagram for explaining a state where a visibility determination unit calculates color information from image information acquired from an endoscopic image and highlight display setting information and determines a visibility of highlight display.
Fig. 9 is an example of a display screen in a case where a display control unit performs highlight display of a region-of-interest and display of notification information.
Fig. 10 is a flowchart illustrating a series of flows of a region-of-interest detection mode.
Fig. 11 is an explanatory diagram illustrating display states, and is an explanatory diagram illustrating an example (A) of detecting a lesion portion from an endoscopic image and an example (B) of superimposing a figure as a highlight display on the endoscopic image.
Fig. 12 is an explanatory diagram illustrating display states, and is an explanatory diagram illustrating an example (A) in which a color difference is decreased and thus a visibility is decreased and an example (B) in which notification information is displayed.
Fig. 13 is an explanatory diagram illustrating display states according to a second embodiment, and is an explanatory diagram illustrating an example in which identification information is displayed in a case where the visibility is low (A) and in a case where the visibility is high (B).
Fig. 14 is an explanatory diagram illustrating display states according to a third embodiment, and is an explanatory diagram illustrating an example in which an identification figure is displayed in a case where the visibility is low (A) and in a case where the visibility is high (B).
Fig. 15 is an explanatory diagram illustrating a display state according to a fourth embodiment, and is an explanatory diagram illustrating an example in which a main image and a sub image are displayed on one display screen and an identification figure is superimposed on the sub image.
Fig. 16 is an explanatory diagram illustrating display states according to a fifth embodiment, and is an explanatory diagram illustrating an example (A) in which an object other than a detection target exists in the inside of a figure and an example (B) in which an identification figure is displayed in the case.
Fig. 17 is an explanatory diagram illustrating, as a modification example, an example in which highlight display includes four L-shaped figures surrounding a lesion portion.
Fig. 18 is an explanatory diagram illustrating a display state according to a sixth embodiment.
Fig. 19 is an explanatory diagram illustrating a display state according to a seventh embodiment.

### DESCRIPTION OF THE PREFERRED EMBODIMENTS

### [First Embodiment]

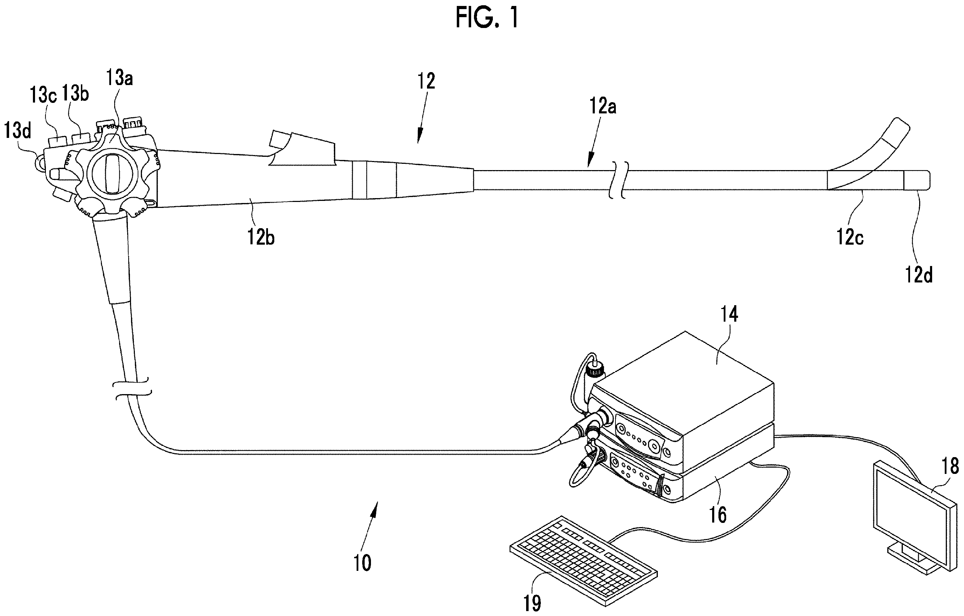

As illustrated in Fig. 1, an endoscope system 10 includes an endoscope 12, a light source device 14, a processor device 16, a monitor (display unit) 18, and a console 19. The endoscope 12 is optically connected to the light source device 14, and is electrically connected to the processor device 16. The endoscope 12 includes an insertion part 12a to be inserted into a body of a subject, an operating part 12b provided at a proximal end portion of the insertion part 12a, and a bendable part 12c and a tip part 12d provided on a distal end side of the insertion part 12a. In a case where an angle knob 13a of the operating part 12b is operated, a bending operation of the bendable part 12c is performed. By the bending operation, the tip part 12d is directed in a desired direction.

The tip part 12d includes an illumination window, an observation window, an air supply/water supply nozzle, and a forceps outlet on a distal end surface (all not illustrated). The illumination window is for irradiating an observation portion with an illumination light beam. The observation window is for taking in a light beam from the observation portion. The air supply/water supply nozzle is for cleaning the illumination window and the observation window. The forceps outlet is for performing various treatments using a forceps and a treatment tool such as an electric scalpel.

In addition to the angle knob 13a, the operating part 12b includes a still image acquisition unit 13b used for a still image acquisition operation, a mode switching unit 13c used for an observation mode switching operation, and a zoom operating part 13d used for a zoom magnification changing operation. The still image acquisition unit 13b can perform a freeze operation for displaying a still image of an observation target on the monitor 18 and a release operation for storing the still image in a storage.

The endoscope system 10 has a normal mode, a special mode, and a region-of-interest detection mode as observation modes. In a case where the observation mode is the normal mode, a normal light beam obtained by combining light beams having a plurality of colors at a normal-mode light quantity ratio Lc is emitted. Further, in a case where the observation mode is the special mode, a special light beam obtained by combining light beams having a plurality of colors at a special-mode light quantity ratio Ls is emitted.

Further, in a case where the observation mode is the region-of-interest detection mode, an illumination light beam for the region-of-interest detection mode is emitted. In the present embodiment, as the illumination light beam for the region-of-interest detection mode, the normal light beam is emitted. On the other hand, the special light beam may be emitted.

The processor device 16 is electrically connected to the monitor 18 and the console 19. The monitor 18 outputs and displays an image of the observation target, information related to the image, and the like. The console 19 functions as a user interface that receives an input operation such as designation of a region-of-interest (ROI) or function setting.

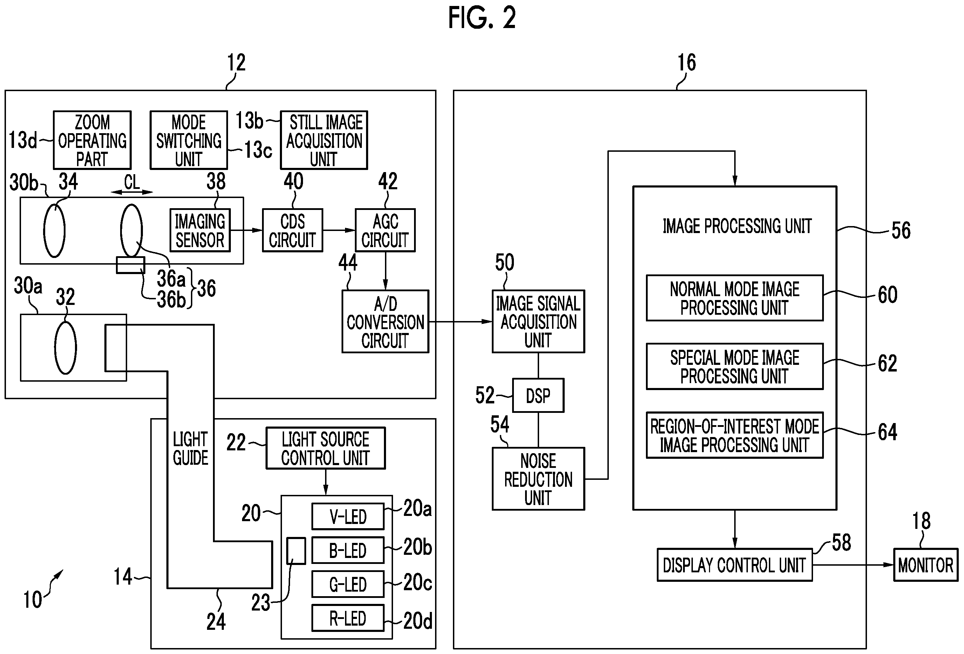

As illustrated in Fig. 2, the light source device 14 includes a light source unit 20 that emits an illumination light beam used for illuminating an observation target, and a light source control unit 22 that controls the light source unit 20. The light source unit 20 is a semiconductor light source such as a light emitting diode (LED) which emits light beams having a plurality of colors. The light source control unit 22 controls a light emission amount of the illumination light beams by turning ON/OFF the LEDs or adjusting a drive current or a drive voltage of the LEDs. Further, the light source control unit 22 controls a wavelength range of the illumination light beams by changing an optical filter or the like.

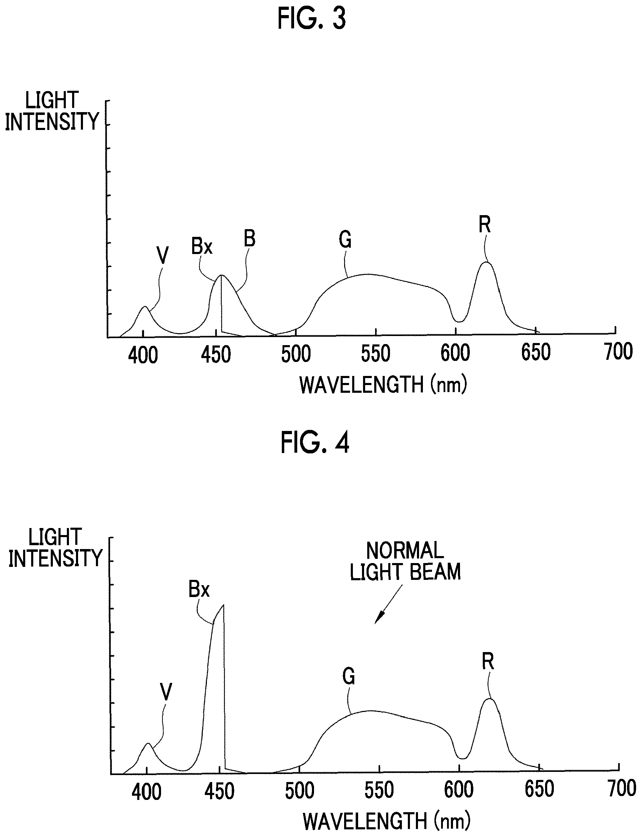

In the first embodiment, the light source unit 20 includes four-color LEDs of a violet light emitting diode (V-LED) 20a, a blue light emitting diode (B-LED) 20b, a green light emitting diode (G-LED) 20c, and a red light emitting diode (R-LED) 20d and a wavelength cut filter 23. As illustrated in Fig. 3, the V-LED 20a emits a violet light beam V in a wavelength range of 380 nm to 420 nm.

The B-LED 20b emits a blue light beam B in a wavelength range of 420 nm to 500 nm. In the blue light beams B emitted from the B-LED 23b, at least a light beam having a wavelength longer than a peak wavelength of 450 nm is cut by the wavelength cut filter 23. Thereby, the blue light beam Bx passing through the wavelength cut filter 23 is within a wavelength range of 420 nm to 460 nm. The reason why the light beam in a wavelength range including wavelengths longer than 460 nm is cut in this way is that the light beam in a wavelength range including wavelengths longer than 460 nm causes a decrease in vascular contrast of a blood vessel as an observation target. The wavelength cut filter 23 may dim the light beam in a wavelength range including wavelengths longer than 460 nm instead of cutting the light beam in a wavelength range including wavelengths longer than 460 nm.

The G-LED 20c emits a green light beam G in a wavelength range of 480 nm to 600 nm. The R-LED 20d emits a red light beam R in a wavelength range of 600 nm to 650 nm. In the light beams emitted from the LEDs 20a to 20d, central wavelengths and peak wavelengths may be the same, or may be different from each other.

The light source control unit 22 adjusts a light emission timing, a light emission period, a light emission amount, and a spectral spectrum of the illumination light beams by independently controlling ON/OFF of each of the LEDs 20a to 20d, a light emission amount of each of the LEDs in an ON state, or the like. The light source control unit 22 controls ON/OFF of the LEDs depending on the observation mode. The reference brightness can be set by a brightness setting unit of the light source device 14, the console 19, or the like.

In a case of the normal mode or the region-of-interest detection mode, the light source control unit 22 turns on all the V-LED 20a, the B-LED 20b, the G-LED 20c, and the R-LED 20d. At that time, as illustrated in Fig. 4, a light quantity ratio Lc between the violet light beam V, the blue light beam B, the green light beam G, and the red light beam R is set such that a peak of a light intensity of the blue light beam Bx is higher than a peak of a light intensity of any one of the violet light beam V, the green light beam G, and the red light beam R. Thereby, in the normal mode or the region-of-interest detection mode, the light beams for the normal mode or the region-of-interest detection mode that have the plurality of colors and include the violet light beam V, the blue light beam Bx, the green light beam G, and the red light beam R are emitted from the light source device 14, as the normal light beams. The normal light beam is almost white because the normal light beam has an intensity of a certain level or higher from a blue wavelength range to a red wavelength range.

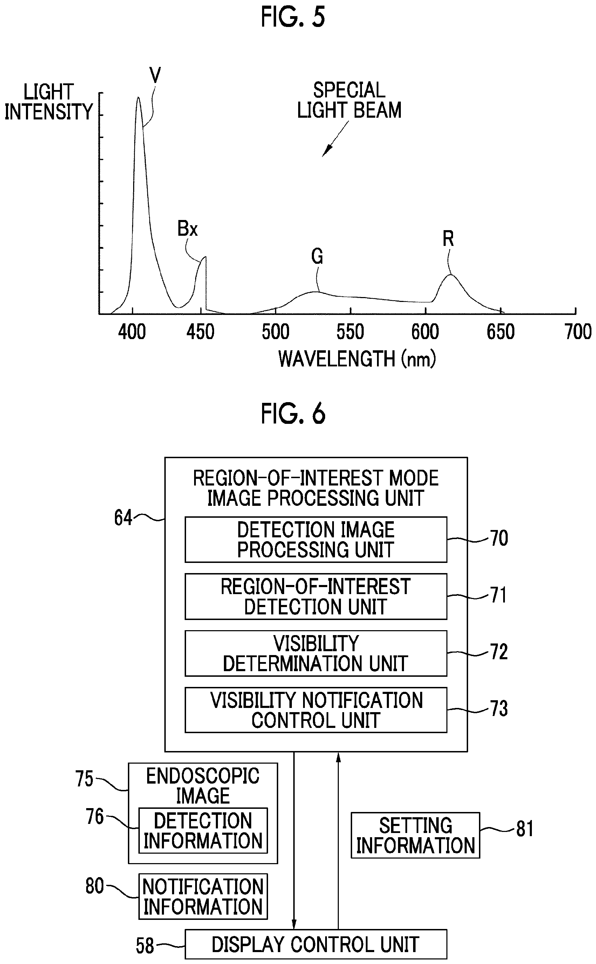

In a case of the special mode, the light source control unit 22 turns on all the V-LED 20a, the B-LED 20b, the G-LED 20c, and the R-LED 20d. At that time, as illustrated in Fig. 5, a light quantity ratio Ls between the violet light beam V, the blue light beam B, the green light beam G, and the red light beam R is set such that a peak of a light intensity of the violet light beam V is higher than a peak of a light intensity of any one of the blue light beam Bx, the green light beam G, and the red light beam R. Further, the peaks of the light intensities of the green light beam G and the red light beam R are set to be lower than the peaks of the light intensities of the violet light beam V and the blue light beam Bx. Thereby, in the special mode, the light beams for the special mode that have the plurality of colors and include the violet light beam V, the blue light beam Bx, the green light beam G, and the red light beam R are emitted from the light source device 14, as the special light beams. The special light beam is bluish because a proportion of the violet light beams V is high. The special light beam may not include light beams having all four colors, and may include at least a light beam from a one-color LED among the four-color LEDs 20a to 20d. Further, preferably, the special light beam has a main wavelength range, for example, a peak wavelength or a central wavelength within a range of 450 nm or lower.

As illustrated in Fig. 2, the illumination light beam emitted by the light source unit 20 is incident on a light guide 24 inserted into the insertion part 12a via an optical path coupling unit (not illustrated) formed by a mirror, a lens, and the like. The light guide 24 is incorporated in the endoscope 12 and the universal cord, and propagates the illumination light beam to the tip part 12d of the endoscope 12. The universal cord is a cord that connects the endoscope 12, the light source device 14, and the processor device 16. As the light guide 24, a multi-mode fiber can be used. As an example, for the light guide 24, a fine fiber cable having a core diameter of 105 µm, a clad diameter of 125 µm, and a diameter of ϕ0.3 mm to ϕ0.5 mm including a protective layer serving as an outer skin can be used.

An illumination optical system 30a and an imaging optical system 30b are provided at the tip part 12d of the endoscope 12. The illumination optical system 30a includes an illumination lens 32. The observation target is illuminated with the illumination light beam propagating through the light guide 24 via the illumination lens 32. The imaging optical system 30b includes an objective lens 34, a magnification optical system 36, and an imaging sensor 38 (corresponding to "imaging unit" according to the present invention). Various light beams such as a reflected light beam, a scattered light beam, and a fluorescent light beam from the observation target are incident on the imaging sensor 38 via the objective lens 34 and the magnification optical system 36. Thereby, an image of the observation target is formed on the imaging sensor 38.

The magnification optical system 36 includes a zoom lens 36a that magnifies the observation target and a lens driving unit 36b that moves the zoom lens 36a in an optical axis direction CL. The zoom lens 36a is freely moved between a telephoto end and a wide end according to zoom control by the lens driving unit 36b. Thereby, the observation target imaged on the imaging sensor 38 is enlarged or reduced.

The imaging sensor 38 is a color imaging sensor that images the observation target irradiated with the illumination light beam. For each pixel of the imaging sensor 38, any one of an R (red) color filter, a G (green) color filter, and a B (blue) color filter is provided. The imaging sensor 38 receives light beams including a violet light beam to a blue light beam from a B pixel for which the B color filter is provided, receives a green light beam from a G pixel for which the G color filter is provided, and receives a red light beam from an R pixel for which the R color filter is provided. In addition, an image signal of each of RGB colors is output from each color pixel. The imaging sensor 38 transmits the output image signal to a CDS circuit 40.

In the normal mode or the region-of-interest detection mode, the imaging sensor 38 outputs a Bc image signal from the B pixel, outputs a Gc image signal from the G pixel, and outputs an Rc image signal from the R pixel by imaging the observation target illuminated with the normal light beam. Further, in the special mode, the imaging sensor 38 outputs a Bs image signal from the B pixel, outputs a Gs image signal from the G pixel, and outputs an Rs image signal from the R pixel by imaging the observation target illuminated with the special light beam.

As the imaging sensor 38, a charge coupled device (CCD) imaging sensor, a complementary metal-oxide semiconductor (CMOS) imaging sensor, or the like can be used. Further, instead of the imaging sensor 38 provided with RGB primary color filters, a complementary color imaging sensor provided with complementary color filters for C (cyan), M (magenta), Y (yellow), and G (green) may be used. In a case where a complementary color imaging sensor is used, image signals of four colors of CMYG are output. Thus, by converting the image signals of four colors of CMYG into image signals of three colors of RGB by complementary-color-to-primary-color conversion, an image signal of each of RGB colors can be obtained as in the imaging sensor 38. Further, instead of the imaging sensor 38, a monochrome sensor without a color filter may be used.

The CDS circuit 40 performs correlated double sampling (CDS) on the analog image signal received from the imaging sensor 38. The image signal that passes through the CDS circuit 40 is input to an AGC circuit 42. The AGC circuit 40 performs automatic gain control (AGC) on the input image signal. An analog to digital (A/D) conversion circuit 44 converts the analog image signal that passes through the AGC circuit 42 into a digital image signal. The A/D conversion circuit 44 inputs the digital image signal after the A/D conversion to the processor device 16.

As illustrated in Fig. 2, the processor device 16 includes an image signal acquisition unit 50, a digital signal processor (DSP) 52, a noise reduction unit 54, an image processing unit 56, and a display control unit 58.

The processor device 16 functions as a medical image processing apparatus. As will be described later, the image processing unit 56 acquires an endoscopic image, and detects a region-of-interest in the observation target from the endoscopic image. The display control unit 58 performs highlight display of the region-of-interest on the endoscopic image 75.

The image signal acquisition unit 50 acquires, from the endoscope 12, a digital image signal corresponding to the observation mode. In a case of the normal mode or the region-of-interest detection mode, a Bc image signal, a Gc image signal, and an Rc image signal are acquired. In a case of the special mode, a Bs image signal, a Gs image signal, and an Rs image signal are acquired. In a case of the region-of-interest detection mode, when the observation target is illuminated with the normal light beam, a Bc image signal, a Gc image signal, and an Rc image signal for one frame are acquired, and when the observation target is illuminated with the special light beam, a Bs image signal, a Gs image signal, and an Rs image signal for one frame are acquired.

The DSP 52 performs various signal processing such as defect correction processing, offset processing, DSP gain correction processing, linear matrix processing, gamma conversion processing, and demosaicing processing on the image signal acquired by the image signal acquisition unit 50. The defect correction processing corrects a signal of a defective pixel of the imaging sensor 38. The offset processing sets an accurate zero level by removing a dark current component from the image signal after the defect correction processing. The DSP gain correction processing adjusts a signal level by multiplying the image signal after the offset processing by a specific DSP gain.

The linear matrix processing enhances a color reproducibility of the image signal after the DSP gain correction processing. The gamma conversion processing adjusts brightness and chroma saturation of the image signal after the linear matrix processing. The demosaicing processing (also referred to as isotropic processing or synchronization processing) is performed on the image signal after the gamma conversion processing, and thus a signal of a color which is insufficient in each pixel is generated by interpolation. By the demosaicing processing, all the pixels have signals of each color of RGB colors. The noise reduction unit 54 reduces noise by performing noise reduction processing by, for example, a movement average method, a median filter method, or the like on the image signal after the demosaicing processing and the like by the DSP 52. The image signal after the noise reduction is input to the image processing unit 56.

The image processing unit 56 includes a normal mode image processing unit 60, a special mode image processing unit 62, and a region-of-interest detection mode image processing unit 64. The normal mode image processing unit 60 operates in a case where the normal mode is set, and performs color conversion processing, color enhancement processing, and structure enhancement processing on the Bc image signal, the Gc image signal, and the Rc image signal which are received. In the color conversion processing, color conversion processing including 3×3 matrix processing, gradation transformation processing, three-dimensional look up table (LUT) processing, and the like is performed on the RGB image signal.

The color enhancement processing is performed on the RGB image signal after the color conversion processing. The structure enhancement processing is processing for enhancing a structure of the observation target, and is performed on the RGB image signal after the color enhancement processing. A normal image can be obtained by performing various image processing and the like as described above. Since the normal image is an image obtained based on the normal light beam in which the violet light beam V, the blue light beam Bx, the green light beam G, and the red light beam R are well balanced, the normal image has a natural hue. The normal image is input to the display control unit 58.

The special mode image processing unit 62 operates in a case where the special mode is set. The special mode image processing unit 62 performs color conversion processing, color enhancement processing, and structure enhancement processing on the Bs image signal, the Gs image signal, and the Rs image signal which are received. The processing contents of the color conversion processing, the color enhancement processing, and the structure enhancement processing are the same as the processing contents in the normal mode image processing unit 60. A special image can be obtained by performing various image processing as described above. The special image is an image obtained based on the special light beam in which the light emission amount of the violet light beam V is larger than the light emission amounts of the blue light beam Bx, the green light beam G, and the red light beam R of other colors, the violet light beam having a high absorption coefficient of hemoglobin in a blood vessel. Thus, a resolution of a vascular structure or a ductal structure is higher than a resolution of another structure. The special image is input to the display control unit 58.

The region-of-interest detection mode image processing unit 64 operates in a case where the region-of-interest detection mode is set. As illustrated in Fig. 6, the region-of-interest detection mode image processing unit 64 includes a detection image processing unit 70, a region-of-interest detection unit 71, a visibility determination unit 72, and a visibility notification control unit 73. The detection image processing unit 70 sequentially acquires an endoscopic image 75 by performing the same image processing as the processing in the normal mode image processing unit 60, such as color conversion processing, on the Bc image signal, the Gc image signal, and the Rc image signal which are received.

The region-of-interest detection unit 71 analyzes the endoscopic image 75, and performs region-of-interest detection processing for detecting a region-of-interest in the observation target. In the present embodiment, the region-of-interest detection unit 71 detects, as a region-of-interest, a lesion portion (for example, a tumor, inflammation, or the like) in the observation target. In this case, the region-of-interest detection unit 71 first divides the endoscopic image 75 into a plurality of small regions, for example, square regions for the number of pixels. Next, an image feature amount is calculated from the divided endoscopic image 75. Subsequently, based on the calculated feature amount, recognition processing as to whether or not each small region is a lesion portion is performed. As the recognition processing, preferably, a machine learning algorithm such as a convolutional neural network or deep learning is used.

Further, the feature amount calculated from the endoscopic image 75 by the region-of-interest detection unit 71 is preferably a value obtained from a shape or a color of a predetermined portion of the observation target or a value obtained from the shape and the color. For example, as the feature amount, preferably, at least one of a density of a blood vessel, a shape of a blood vessel, the number of branches of a blood vessel, a thickness of a blood vessel, a length of a blood vessel, a tortuosity of a blood vessel, a reaching depth of a blood vessel, a shape of a duct, a shape of an opening of a duct, a length of a duct, a tortuosity of a duct, or color information, or a value obtained by combining two or more of these values is used.

Finally, a group of small regions identified as the same type is extracted as one lesion portion. The region-of-interest detection unit 71 associates information of the extracted lesion portion such as position information, a size, and a lesion type of the lesion portion with the endoscopic image 75, as detection information 76. The region-of-interest detection mode image processing unit 64 outputs the endoscopic image 75 associated with the detection information 76 to the display control unit 58.

The display control unit 58 performs display control for displaying the image or data from the image processing unit 56 on the monitor 18. In a case where the normal mode is set, the display control unit 58 controls to display the normal image on the monitor 18. In a case where the special mode is set, the display control unit 58 controls to display the special image on the monitor 18.

In a case where the region-of-interest detection mode is set, the display control unit 58 performs highlight display of the region-of-interest detected by the region-of-interest detection unit 71 on the endoscopic image 75. In a case of performing highlight display of the region-of-interest, the display control unit 58 first sets a highlight region for highlighting the region-of-interest based on the endoscopic image 75 output from the region-of-interest detection mode image processing unit 64 and the detection information 76 associated with the endoscopic image 75.

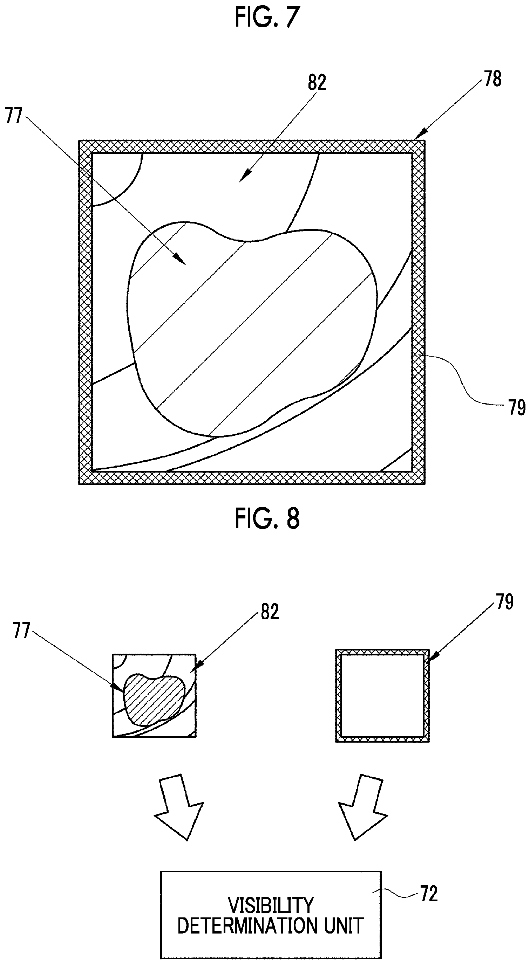

As illustrated in Fig. 7, the display control unit 58 sets a highlight region 78 that has an area larger than the lesion portion 77 and includes the lesion portion 77, based on the detection information 76 such as the position, the size, and the type of the lesion portion 77. In the present embodiment, a square region is set as the highlight region 78. The highlight region 78 has, for example, a square outer circumference that is set at a predetermined interval from an outer circumference of the lesion portion 77. The highlight region 78 is not limited thereto, and may be set to a square in contact with the outer circumference of the lesion portion 77.

The display control unit 58 performs highlight display of the highlight region 78 which is set as described above. That is, the display control unit 58 superimposes and displays a figure as a highlight display at a position of the highlight region 78 in the endoscopic image 75. In the present embodiment, the display control unit 58 displays a square-frame-shaped (frame-shaped) figure 79 surrounding the lesion portion 77 in accordance with the position of the highlight region 78. After the highlight region 78 is set, the display control unit 58 resets the highlight region 78 according to a change amount of the lesion portion 77 in the endoscopic image 75, and displays the figure 79 in accordance with the position of the reset highlight region 78.

In addition, the figure 79 as the highlight display has a display form different from display forms of other portions of the endoscopic image 75. The display control unit 58 displays the figure 79, for example, in a color having a hue different from a color which is generally and mostly included in the endoscopic image. In addition, the color of the figure 79 may be set according to an input operation by a user.

The display control unit 58 outputs setting information 81 of the figure 79 as the highlight display to the image processing unit 56. The setting information 81 includes position information, color information, and the like of the figure 79 with respect to the endoscopic image 75. The setting information 81 is tagged with the information of the original endoscopic image 75 in which the lesion portion 77 is detected.

The visibility determination unit 72 determines a visibility of the highlight display from the image information acquired from the endoscopic image 75 in which the lesion portion 77 is detected and the highlight display setting information 81 which is set by the display control unit 58, and calculates, as a determination result, a numerical index value. In the present embodiment, the visibility determination unit 72 calculates pieces of color information from the image information and the highlight display setting information 81, and calculates, as an index value, a color difference between the endoscopic image 75 and the figure 79 from the pieces of color information, the image information being acquired from the endoscopic image 75 in which the lesion portion 77 is detected by the region-of-interest detection unit 71. The color information indicates information related to colors such as hue, brightness, and chroma saturation.

As illustrated in Fig. 8, in a case of calculating the color information from the image information acquired from the endoscopic image 75, the visibility determination unit 72 calculates, as the color information, an average value in a range 82 (also refer to Fig. 7) surrounded by the inside of the highlight display including the lesion portion 77, specifically, the figure 79 in the endoscopic image 75. As described above, since the setting information 81 includes the position information of the figure 79, it is possible to calculate the color information by cutting out the range 82 surrounded by the figure 79 from the endoscopic image 75 based on the position information. On the other hand, in a case of calculating the color information from the highlight display setting information, an average value of the figure 79 is calculated as the color information. The visibility determination unit 72 calculates a color difference between the endoscopic image 75 and the figure 79 from the pieces of color information.

In the calculation of the color difference by the visibility determination unit 72, for example, the color difference is obtained by a color difference equation according to CIEDE2000 defined in JIS Z 8730 7.3. By using a calculation method standardized in this way, it is possible to obtain a color difference in accordance with human visual characteristics. In a case where the color difference equation according to the CIEDE 2000 is used, as the color information for obtaining the color difference, information on a CIELab color space is used, the color space including an L component indicating brightness, a component indicating a level of red or green, and a b component indicating a level of yellow or blue.

The calculation method for obtaining the color difference is not limited to the above-described method, and any calculation method considering human vision may be used. For example, the color difference may be calculated using the Euclidean distance (also referred to as CIE76) in the CIELab color space.

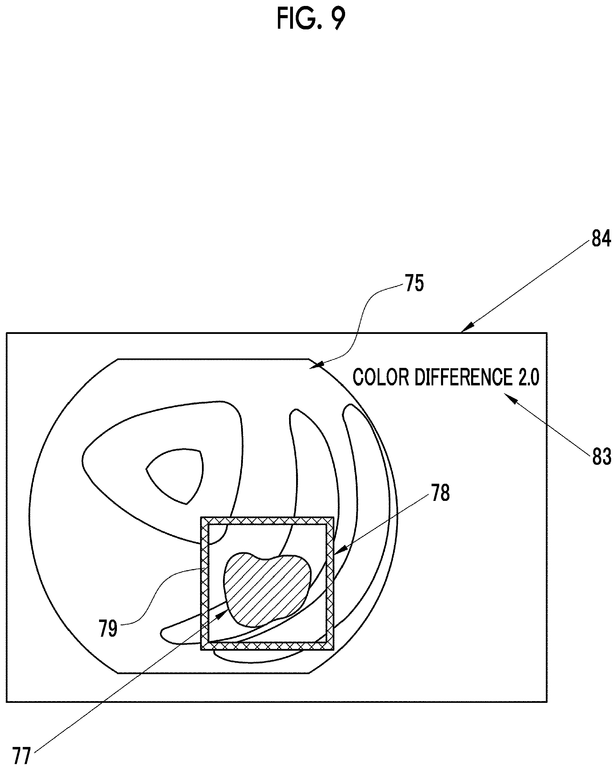

The visibility notification control unit 73 notifies the user of the determination result determined by the visibility determination unit 72. As illustrated in Fig. 9, the visibility notification control unit 73 outputs, as notification information 83, the color difference as the determination result calculated as described above to the display control unit 58, and displays the color difference on a display screen 84 of the monitor 18. In the present embodiment, in a case where the color difference is equal to or smaller than a preset first threshold value, the visibility notification control unit 73 outputs, as the notification information 83, information of the color difference to the display control unit 58.

In the visibility notification control unit 73, for example, it is assumed that the preset first threshold value is set to 2.0. According to the JIS standard, in a case where a color difference is around 1, the color difference is defined as a level at which a difference can be determined when two colors are compared side by side, and in a case where a color difference is 2 to 3, the color difference is defined as a level at which a difference can be seen when two colors are separated and compared. Since the first threshold value is set to 2.0 based on the JIS standard as described above, the visibility notification control unit 73 can notify the user that the visibility is decreased.

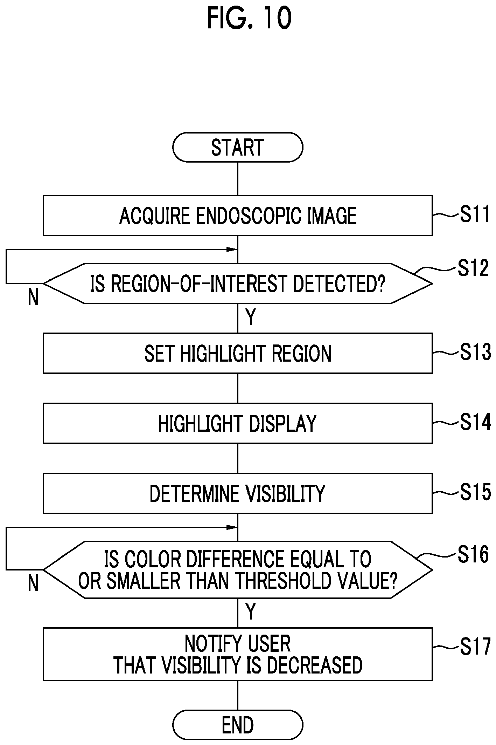

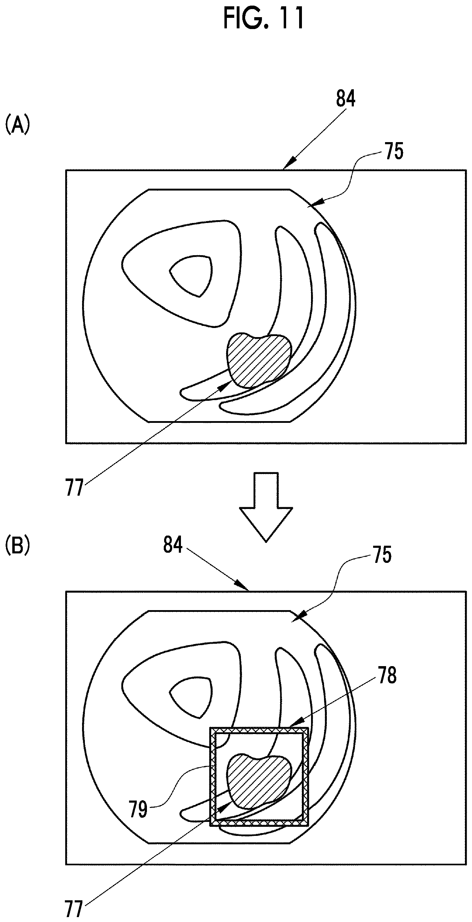

Hereinafter, in the region-of-interest detection mode, a process in which the image processing unit 56 and the display control unit 58 determine the visibility of the highlight display and display the determination result on the display screen 84 of the monitor 18 will be described with reference to a flowchart illustrated in Fig. 10 and an explanatory diagram illustrated in Fig. 11. A doctor as a user switches the observation mode to the region-of-interest detection mode by operating the mode switching unit 13c. Thereby, an observation target is illuminated with an illumination light beam for the region-of-interest detection mode. The imaging sensor 38 images the observation target illuminated with the illumination light beam for the region-of-interest detection mode, and thus an endoscopic image 75 is acquired. In a case where the observation mode is switched to the region-of-interest detection mode, the display control unit 58 sequentially acquires the endoscopic images 75 (S11), and displays the endoscopic images in real time on the display screen 84 of the monitor 18.

During a period for which the endoscopic images are displayed in real time in the region-of-interest detection mode, the region-of-interest detection unit 71 performs region-of-interest detection processing for detecting a region-of-interest in the observation target on the acquired endoscopic image 75. In a case where a region-of-interest is detected (Y in S12), the region-of-interest detection unit 71 outputs the detection information 76 associated with the endoscopic image 75.

In addition, as illustrated in Fig. 11(A), in a case where a lesion portion 77 as a region-of-interest is detected in the observation target, that is, in a case where the detection information 76 is associated with the endoscopic image 75, the display control unit 58 sets a highlight region 78 by using the detection information 76 associated with the endoscopic image 75, particularly, information on a position and a size of the lesion portion 77 (S13).

After the highlight region 78 is set, as illustrated in Fig. 11(B), the display control unit 58 superimposes and displays a figure 79 as a highlight display at a position of the highlight region 78 in the endoscopic image 75 (S14), and outputs the setting information 81 of the figure 79 to the image processing unit 56. In addition, in Fig. 7 to Fig. 9, Fig. 11, and Fig. 12, for convenience of illustration, a difference in color between the figure 79 and other portions in the endoscopic image 75 is represented by the presence or absence of shading. On the other hand, in a case where a lesion portion 77 is not detected in the observation target (N in S12), highlight display is not performed as a matter of course.

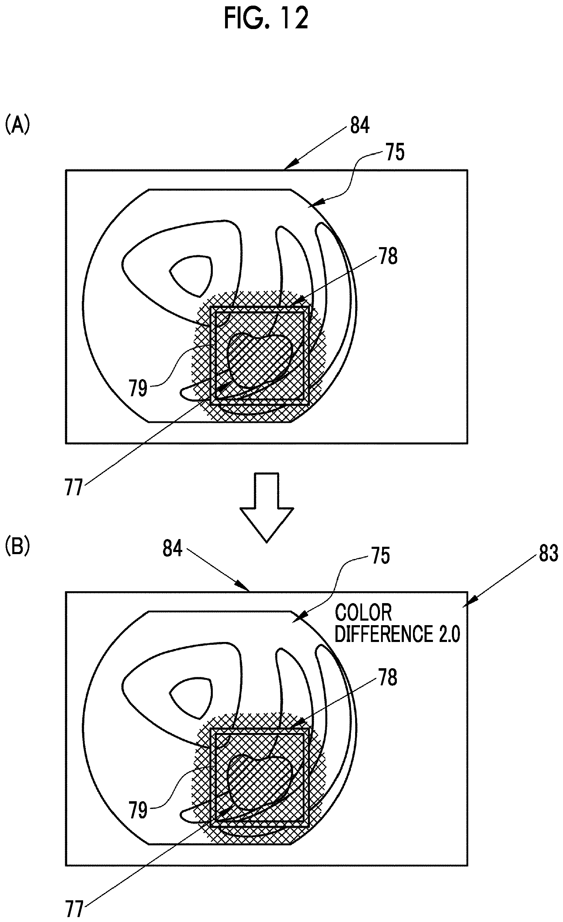

Since the setting information 81 of the figure 79 is tagged with the information of the original endoscopic image 75 in which the lesion portion 77 is detected, the visibility determination unit 72 reads the original endoscopic image 75, calculates pieces of color information from the image information acquired from the endoscopic image 75 and the setting information 81 of the figure 79, and determines a visibility of the highlight display (S15). In the visibility determination, as described above, the color difference calculated from the pieces of color information is compared with the first threshold value. As illustrated in Fig. 12(A), in a case where the figure 79 as the highlight display is displayed, the figure 79 may be assimilated with surroundings or may be less conspicuous with respect to surrounding portions, depending on a color of a subject in the endoscopic image 75, the presence or absence of an object existing in the subject, and the like. As a result, the visibility may be decreased. In such a case, in general, a value of the color difference between the endoscopic image 75 and the figure 79 also decreases.

In a case where the color difference is equal to or smaller than the first threshold value (Y in S16), as illustrated in Fig. 12(B), information of the color difference is output to the display control unit 58, as the notification information 83. The display control unit 58 notifies the user that the visibility is decreased by displaying the notification information 83 on the display screen 84 (S17). In a case where the color difference exceeds the first threshold value (N in S16), the visibility determination unit 72 does not perform notification.

As described above, the visibility of the highlight display in the endoscopic image 75 is determined, and in a case where the visibility of the highlight display is decreased, notification is performed. Therefore, a doctor as a user can recognize a decrease in visibility of the highlight display, and it is possible to avoid a state where the user does not notice the region-of-interest such as the lesion portion.

### [Second Embodiment]

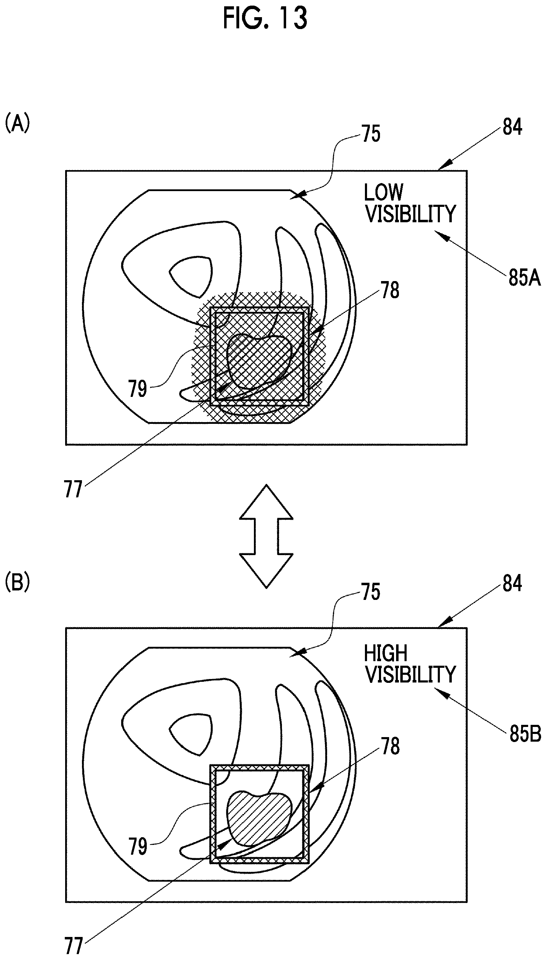

In the first embodiment, an example in which the information of the color difference is displayed as a determination result of the visibility on the display screen is given. On the other hand, the present invention is not limited thereto, and identification information may be displayed according to the index value as the determination result. Fig. 13 illustrates an example in which pieces of identification information 85A and 85B according to the color difference as the determination result are displayed on the display screen 84.

The present embodiment is the same as the first embodiment until a process of calculating pieces of color information from the image information acquired from the endoscopic image 75 and the highlight display setting information 81, calculating the color difference between the endoscopic image 75 and the figure 79 from the pieces of color information, and comparing the color difference with the first threshold value. In addition, in a case where the color difference is equal to or smaller than the first threshold value, the visibility determination unit 72 outputs identification information 85A to the display control unit 58. The display control unit 58 notifies the user that the visibility is decreased by displaying the identification information 85A on the display screen 84. In the example illustrated in Fig. 13(A), text information "low visibility" is displayed as the identification information 85A. Thereby, as in the first embodiment, a doctor as a user can recognize a decrease in the visibility of the highlight display.

As a modification example of the present embodiment, the identification information may be displayed not only in a case where the color difference is equal to or smaller than the first threshold value but also in a case where the color difference exceeds the first threshold value, that is, in a case where the visibility is high. As illustrated in Fig. 13(B), in a case where the color difference exceeds the first threshold value, the visibility determination unit 72 outputs identification information 85B to the display control unit 58. The display control unit 58 notifies the user that the visibility is high by displaying the identification information 85B on the display screen 84. In the example illustrated in Fig. 13(B), text information "high visibility" is displayed as the identification information 85B.

In addition, the setting for displaying the identification information according to the color difference is not limited to the two-stage setting of a case where the color difference is equal to or smaller than the first threshold value or a case where the color difference exceeds the first threshold value, and may be set to setting of three or more stages. For example, the visibility determination unit 72 sets three-stage numerical values of a case where the color difference is equal to or smaller than 2.0, a case where the color difference is larger than 2.0 and equal to or smaller than 4.0, and a case where the color difference is larger than 4.0 in advance, and performs determination based on the setting. In addition, in a case where the color difference is equal to or smaller than 2.0, information indicating that the visibility is low is displayed as the identification information on the display screen 84. In a case where the color difference is larger than 2.0 and equal to or smaller than 4.0, information indicating that the visibility is medium is displayed as the identification information on the display screen 84. In a case where the color difference is larger than 4.0, information indicating that the visibility is high is displayed as the identification information on the display screen 84. In this case, preferably, as the identification information, for example, text information such as "low visibility", "medium visibility", and "high visibility" is displayed according to the stage of the color difference.

### [Third Embodiment]

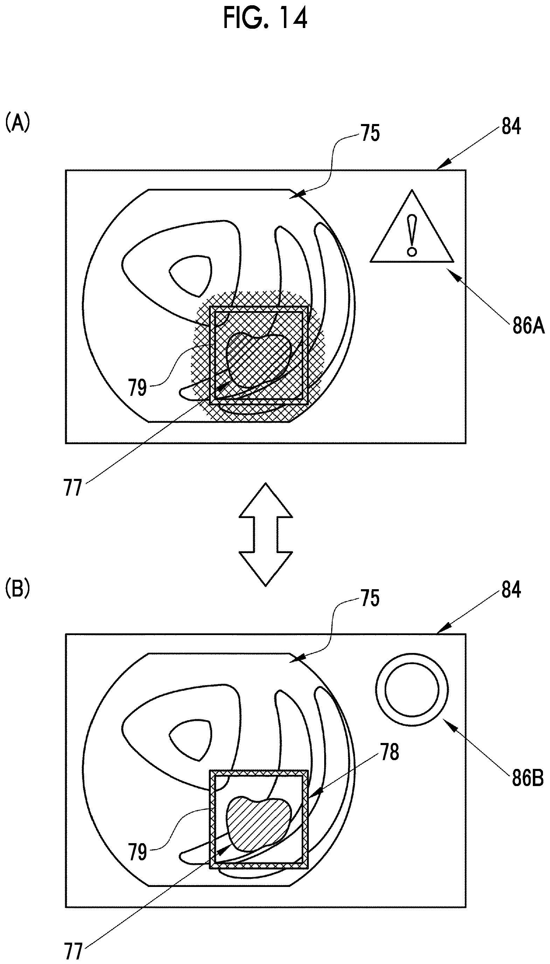

In the second embodiment, an example in which the identification information according to the color difference as the determination result is displayed as the determination result of the visibility is given. On the other hand, the present invention is not limited thereto. As in the example illustrated in Fig. 14, an identification figure according to the color difference as the determination result may be displayed.

The present embodiment is the same as the first embodiment and the second embodiment until a process of calculating pieces of color information from the image information acquired from the endoscopic image 75 and the highlight display setting information 81, calculating the color difference between the endoscopic image 75 and the figure 79 from the pieces of color information, and comparing the color difference with the first threshold value. In addition, in a case where the color difference is equal to or smaller than the first threshold value, information of an icon 86A is output to the display control unit 58. The display control unit 58 notifies the user that the visibility is decreased by displaying the icon 86A as the identification figure on the display screen 84. In the example illustrated in Fig. 14(A), as the icon 86A, a mark imitating a sign indicating that there is a danger is displayed. Thereby, as in the first embodiment and the second embodiment, a doctor as a user can recognize a decrease in the visibility of the highlight display.

As a modification example of the present embodiment, the identification figure may be displayed not only in a case where the color difference is equal to or smaller than the first threshold value but also in a case where the color difference exceeds the first threshold value, that is, in a case where the visibility is high. As illustrated in Fig. 14(B), in a case where the color difference exceeds the first threshold value, the visibility determination unit 72 outputs information of the icon 86B to the display control unit 58. The display control unit 58 notifies the user that the visibility is high by displaying the icon 86B as the identification figure on the display screen 84. In the example illustrated in Fig. 14(B), as the icon 86B, a double circle mark is displayed.

Further, as the setting for displaying the identification information according to the color difference, as in the second embodiment, setting of three or more stages may be set. For example, the visibility determination unit 72 sets three-stage numerical values in advance as in the second embodiment, and performs determination based on the setting. In addition, in a case where the color difference is equal to or smaller than 2.0, information indicating that the visibility is low is displayed as the identification figure on the display screen 84. In a case where the color difference is larger than 2.0 and equal to or smaller than 4.0, information indicating that the visibility is medium is displayed as the identification figure on the display screen 84. In a case where the color difference is larger than 4.0, information indicating that the visibility is high is displayed as the identification figure on the display screen 84. In this case, as the identification figure, preferably, icons having different shapes are displayed according to the stage of the color difference.

### [Fourth Embodiment]

In each of the above-described embodiments, the image obtained by superimposing the highlight display on the endoscopic image is displayed on one display screen, and the notification information or the like is displayed in a non-display region of the endoscopic image. On the other hand, an image obtained by superimposing the notification information or the like may be displayed on a display screen different from the display screen on which the endoscopic image is displayed.

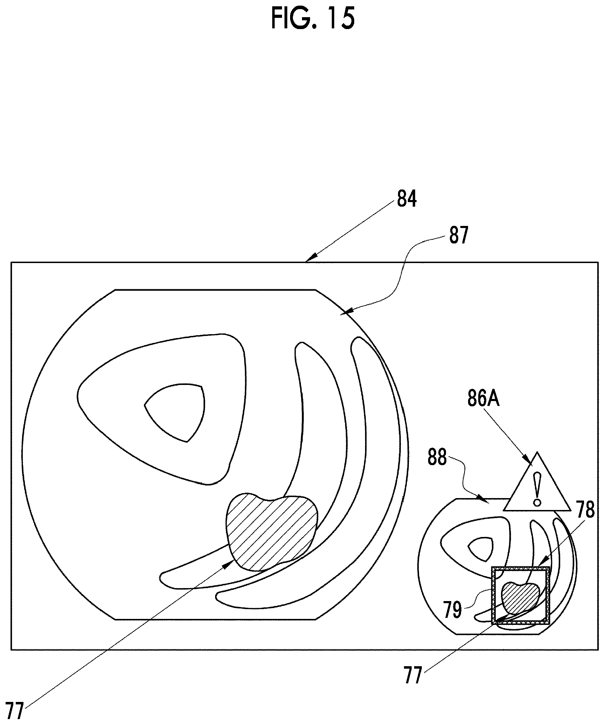

In the example illustrated in Fig. 15, two display screens are displayed side by side on one monitor 18. A normal endoscopic image 87 is displayed as a main image having a large display region, and an image 88 obtained by superimposing highlight display on the endoscopic image is displayed as a sub image having a display region smaller than the display region of the main image. In addition, an icon 86A as the identification information is superimposed and displayed on the image 88 obtained by superimposing the highlight display. In the present embodiment, the normal endoscopic image 87 is the endoscopic image 75 itself acquired by the image processing unit 56 during the region-of-interest detection mode in each of the above-described embodiments, and indicates an image in a state where a figure or the like as a highlight display is not superimposed.

The image 88 as the sub image that is obtained by superimposing the highlight display is an image obtained by superimposing and displaying the figure 79 or the like as the highlight display on the endoscopic image 75 as in each of the above-described embodiments. In addition, as in each of the above-described embodiments, pieces of color information are calculated from the image information acquired from the endoscopic image 75 and the highlight display setting information 81, the color difference between the endoscopic image 75 and the figure 79 is calculated from the pieces of color information, and the color difference is compared with the first threshold value. In addition, in a case where the color difference is equal to or smaller than the first threshold value, information of an icon 86A is output to the display control unit 58. The display control unit 58 notifies the user that the visibility is decreased by further superimposing and displaying an icon 86A on the image 88 obtained by superimposing the highlight display.

The information to be displayed as the determination result of the visibility is not limited to the icon 86A. As in each of the above-described embodiments, the information of the color difference as an index value, the identification information according to the color difference, or the like may be displayed, or different identification information or a different identification figure may be displayed according to the color difference. Further, in the example illustrated in Fig. 15, the two display screens are displayed side by side on one monitor 18. On the other hand, the main image and the sub image may be displayed on different monitors.

In the first embodiment to the fourth embodiment, in a case where the visibility determination unit 72 calculates the color difference between the endoscopic image 75 and the figure 79 as the highlight display, the visibility determination unit 72 calculates the color information from the inside of the figure 79. On the other hand, the present invention is not limited thereto. In the endoscopic image 75, an average value of a portion outside the figure 79, specifically, an average value of a portion excluding the figure 79 and the range 82 surrounded by the figure 79 may be calculated as the color information. As described above, since the setting information 81 includes the position information of the figure 79, it is possible to calculate the color information by cutting out the portion outside the figure 79 from the endoscopic image 75 based on the position information. Thereby, even in a case where the color difference between the figure 79 as the highlight display and the portion outside the figure 79 is decreased, a doctor as a user can recognize a decrease in the visibility of the highlight display.

Further, in the first embodiment to the fourth embodiment, the visibility determination unit 72 uses the preset first threshold value in order to determine the color difference between the endoscopic image 75 and the highlight display. On the other hand, the first threshold value used for the determination is not always the same value, and a weight may be applied to the first threshold value according to a thickness of a line of the highlight display.

For example, the display control unit 58 can change a thickness of a line of the figure 79 as the highlight display according to a size of the region-of-interest, or can change a thickness of a line of the figure 79 according to an input operation of the user. In a case where a thickness of a line of the figure 79 can be changed in this way, the visibility determination unit 72 applies a weight to the first threshold value according to the thickness of the line of the figure 79 when the highlight display setting information is acquired. The weight for the first threshold value is set such that the first threshold value is decreased in inverse proportion to the thickness of the line of the figure 79.

For example, in a case where the thickness of the line of the figure 79 is set to an initial setting value, the first threshold value is set to 2.0. In a case where the thickness of the line of the figure 79 is thicker than the initial setting value, the first threshold value is set to be smaller than 2.0. In a case where the thickness of the line of the figure 79 is thinner than the initial setting value, the first threshold value is set to be larger than 2.0. In the visibility of the highlight display, as the thickness of the line of the figure 79 is thicker, the visibility is higher. Thus, even in a case where the first threshold value is set to be smaller according to the thickness of the line of the figure 79, as in each of the above-described embodiments, a doctor as a user can recognize a decrease in the visibility of the highlight display.

### [Fifth Embodiment]

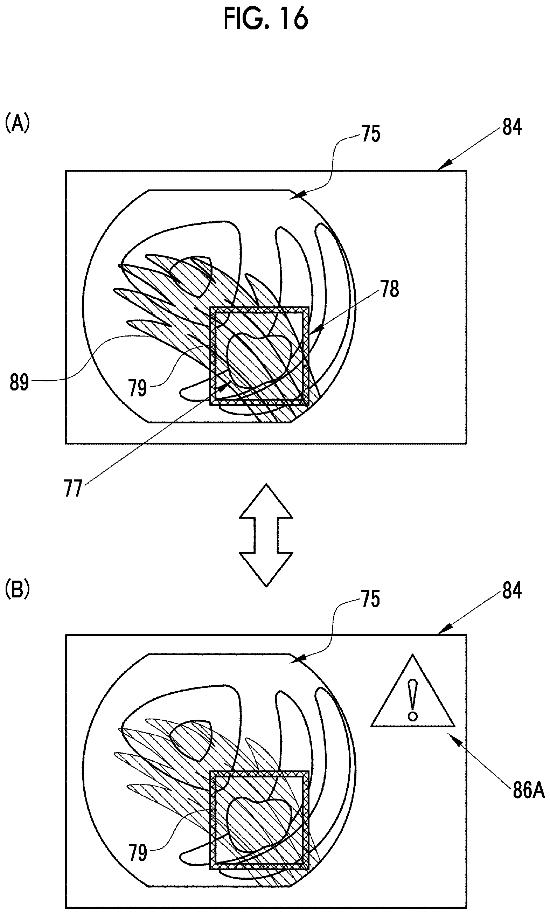

In each of the above-described embodiments, the visibility determination unit 72 determines the visibility by using, as an index value, the color difference calculated from the image information of the endoscopic image 75 and the highlight display setting information. On the other hand, the present invention is not limited thereto. The visibility may be determined based on the presence or absence of an object other than the detection target existing inside the highlight display. In this case, for example, as illustrated in Fig. 16(A), in the endoscopic image 75, as an object 89 or a phenomenon other than the detection target, for example, water, halation, bubbles, coloring agent, or the like other than the lesion portion 77 may enter the inside of the figure 79 as the highlight display. Generally, a portion at which the object 89 or the phenomenon other than the detection target is background-reflected in the endoscopic image 75 has high brightness or high luminance in a case where the color information is acquired. For this reason, in the present embodiment, a brightness value is used as the color information acquired from the endoscopic image. In addition, the present invention is not limited thereto, and a luminance value may be used as the color information acquired from the endoscopic image.

The visibility determination unit 72 compares brightness of each pixel in the range 82 surrounded by the figure 79 with a second threshold value in order to detect that the object 89 or the phenomenon other than the detection target exists inside the figure 79. The second threshold value is set to a value corresponding to high brightness assuming water, halation, bubbles, and the like. Therefore, in a case where the brightness is equal to or higher than the second threshold value, there is a high possibility that the object 89 or the phenomenon other than the detection target is background-reflected.

The visibility determination unit 72 further compares an area ratio of the portion of which the brightness is equal to or higher than the second threshold value to the range 82 surrounded by the figure 79 (ratio of an area of the portion of which the brightness is equal to or higher than the second threshold value to an area of the range 82) with a third threshold value. The third threshold value is set to, for example, an area ratio of 50%, assuming that there are many portions of which the brightness is higher than brightness of the range 82.

In addition, in a case where the area ratio of the portion of which the brightness is equal to or higher than the second threshold value is equal to or higher than the third threshold value, the visibility determination unit 72 determines a state where the object 89 or the phenomenon other than the detection target exists inside the figure 79, that is, that the visibility is decreased, and outputs the information of the icon 86A to the display control unit 58. The display control unit 58 notifies the user that the visibility is decreased by superimposing and displaying the icon 86A on the endoscopic image 75. The information to be displayed as the determination result of the visibility is not limited to the icon 86A. The information of the area ratio of the portion of which the brightness is equal to or higher the second threshold value, the identification information according to the area ratio, or the like may be displayed, or different identification information or a different identification figure may be displayed according to the area ratio.

In each of the above-described embodiments, the figure as the highlight display has a square frame shape. On the other hand, the present invention is not limited thereto. The figure as the highlight display may have a frame shape that can surround the region-of-interest, such as a polygon other than a rectangle (square), a circle, or an ellipse.

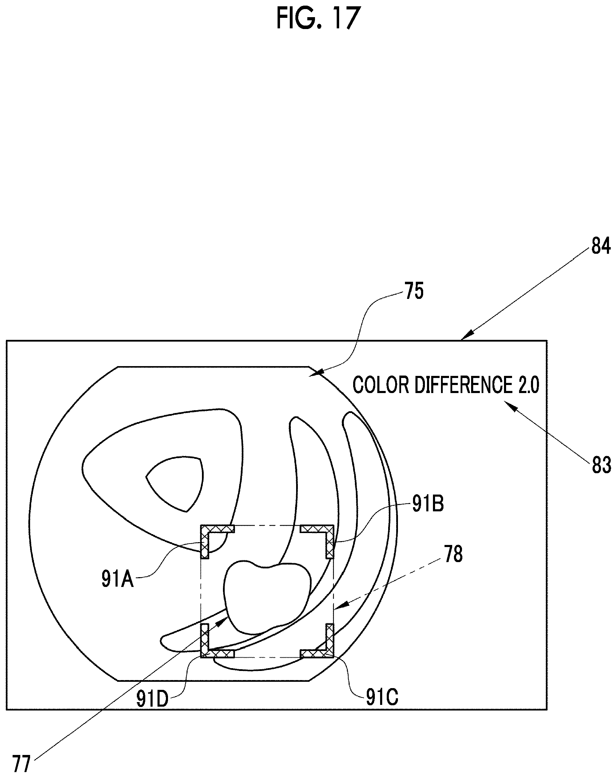

In addition, the shape of the figure as the highlight display is not limited to one frame shape surrounding the region-of-interest, and may include a plurality of shapes. In the example illustrated in Fig. 17, the display control unit 58 disposes, as the highlight display, four L-shaped figures 91A to 91D surrounding the lesion portion 77 on each corner of the highlight region 78. In Fig. 17, a two-dot chain line is illustrated for convenience of explaining arrangement of the L-shaped figures 91A to 91D, and is not actually displayed.

In the example illustrated in Fig. 17, as in the first embodiment to the fourth embodiment, in a case where the color difference calculated from the image information of the endoscopic image 75 and the highlight display setting information is used as the index value in the determination of the visibility, preferably, the visibility determination unit 72 calculates, as color information, an average value of the four L-shaped figures 91A to 91D, calculates a color difference from the color information of the endoscopic image 75 and the color information of the average value of the L-shaped figures 91A to 91D, and compares the color difference with the first threshold value.

The present invention is not limited thereto. The visibility determination unit 72 calculates color information for each of the four L-shaped figures 91A to 91D, calculates a total of four color differences from the color information of the endoscopic image 75 and the color information of each of the L-shaped figures 91A to 91D, and compares the four color differences with the first threshold value. In this case, for example, in a case where any one of the four color differences is equal to or smaller than the first threshold value, it is determined that the visibility is low. In addition, in a case where the color difference is equal to or smaller than the first threshold value, information such as the notification information 83, the identification information, the identification figure, and the like of the color difference is output to the display control unit 58. Thereafter, notification is performed in the same manner as in each of the above-described embodiments.

In addition, in a case where the index value such as the color difference calculated from the image information of the endoscopic image 75 and the setting information of the highlight display is equal to or smaller than the preset first threshold value, or in a case where the area ratio of the portion of which the brightness or the luminance inside the highlight display is equal to or higher than the second threshold value is equal to or larger than the third threshold value, the image processing unit 56 may determine that the visibility is low, perform notification as in each of the above-described embodiments, and automatically store the endoscopic image in which the region-of-interest is detected. Thereby, it is possible to later confirm the endoscopic image in which the visibility is decreased while the region-of-interest is detected. Therefore, it is possible to reliably avoid a state where the user does not notice the region-of-interest such as a lesion portion. In addition, as a storage destination for storing the endoscopic image of which the visibility is determined as being low as described above and in which the region-of-interest is detected, the endoscopic image may be stored in, for example, a storage device provided in the processor device 16 or a server such as a cloud.

In addition, in a case where it is determined that the visibility is low as described above, the image processing unit 56 may not only notify the user that the visibility is low but also perform warning such as outputting of a sound, light emitting of an indicator, or blinking of a portion of a screen.

### [Sixth Embodiment]

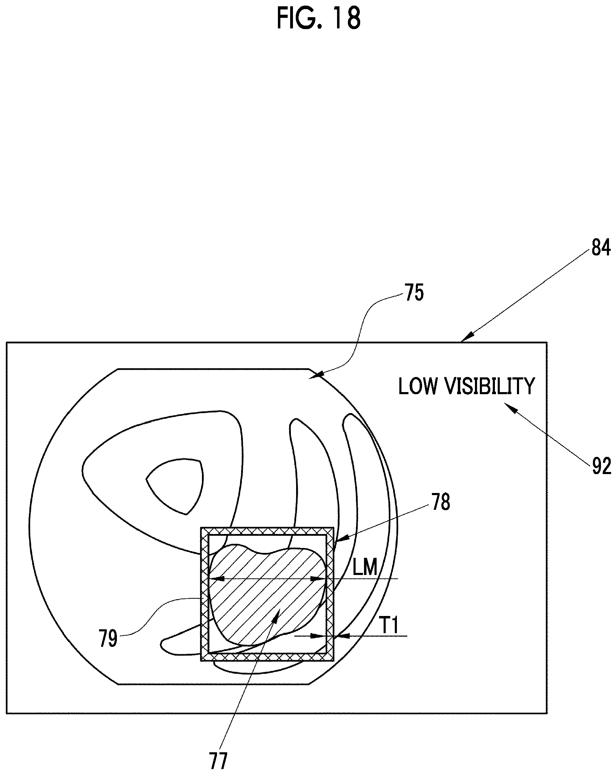

In each of the above-described embodiments, the visibility determination unit 72 determines the visibility based on the information of the color difference, the presence or absence of an object other than the detection target, and the like. On the other hand, the present invention is not limited thereto. The visibility determination unit 72 may determine the visibility from a thickness of a line of the highlight display with respect to the region-of-interest. Fig. 18 is an example of a display screen in a case where the visibility determination unit 72 determines the visibility of the highlight display from a thickness of a line of the highlight display with respect to the region-of-interest.

In the present embodiment, the visibility determination unit 72 calculates, as the index value, a ratio of a thickness T1 of a line of the figure 79 to a maximum dimension LM of the lesion portion 77 detected from the endoscopic image 75 by the region-of-interest detection unit 71. As the maximum dimension LM of the lesion portion 77, for example, a dimension of the largest portion of the lesion portion 77 in any one of an X-axis direction or a Y-axis direction of the endoscopic image 75 is used. In the example illustrated in Fig. 18, the maximum dimension LM is defined as the dimension of the largest portion of the lesion portion 77 in the X-axis direction.

In the determination of the visibility, the visibility determination unit 72 compares the ratio of the thickness T1 of the line of the figure 79 to the maximum dimension LM of the lesion portion 77 described above with a threshold value, and in a case where the ratio is equal to or lower than the threshold value, determines that the visibility is low. In addition, as in each of the above-described embodiments, information such as the notification information, the identification information, and the identification figure is output to the display control unit 58. Thereafter, notification is performed in the same manner as in each of the above-described embodiments. In the example illustrated in Fig. 18, text information "low visibility" is displayed as the identification information 92. As the ratio of the thickness of the line of the figure to the region-of-interest is lower, the visibility is lowered. Therefore, as in each of the above-described embodiments, a doctor as a user can recognize a decrease in the visibility of the highlight display.

### [Seventh Embodiment]

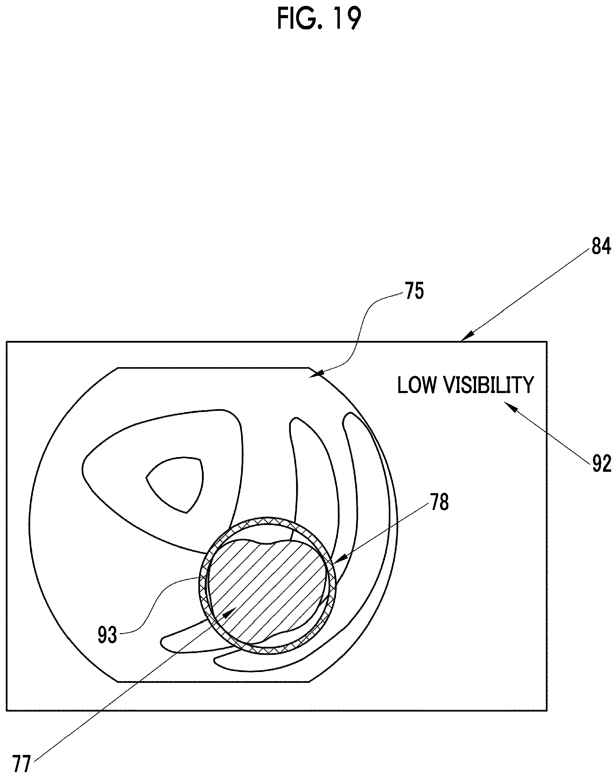

In the sixth embodiment, the visibility determination unit 72 determines the visibility from the thickness of the line of the highlight display with respect to the region-of-interest. On the other hand, the present invention is not limited thereto. The visibility determination unit 72 may display, as the highlight display, a frame-shaped figure surrounding the region-of-interest, and determine the visibility from a similarity of the frame-shaped figure to the region-of-interest. Fig. 19 is an example of a display screen in a case where the visibility of the highlight display is determined from a similarity of the frame-shaped figure to the region-of-interest.

In the present embodiment, the visibility determination unit 72 analyzes a similarity between the shape of the lesion portion 77 detected from the endoscopic image 75 by the region-of-interest detection unit 71 and the frame-shaped figure 93 surrounding the lesion portion 77. The figure 93 is a circular-frame-shaped figure that surrounds the lesion portion 77 and is in contact with a plurality of locations on the outer circumference of the lesion portion 77. In a case where the visibility is determined from the similarity, the visibility determination unit 72 analyzes a similarity between a contour shape of the lesion portion 77 and an inner circumference shape of the figure 93 by, for example, a method such as well-known template matching, compares the similarity with a threshold value, and determines that the visibility is low in a case where the similarity is equal to or higher than the threshold value. In addition, as in each of the above-described embodiments, information such as the notification information, the identification information, and the identification figure is output to the display control unit 58. Thereafter, notification is performed in the same manner as in each of the above-described embodiments. In the example illustrated in Fig. 19, text information "low visibility" is displayed as the identification information 92. As the similarity of the frame-shaped figure to the region-of-interest is higher, the visibility is lowered. Therefore, as in each of the above-described embodiments, a doctor as a user can recognize a decrease in the visibility of the highlight display.

In each of the above-described embodiments, the display control unit 58 superimposes and displays the frame-shaped figure on the position of the highlight region. On the other hand, the present invention is not limited thereto. For the highlight display, the color of the highlight region may be changed. In this case, in a case where the lesion portion 77 as the region-of-interest is detected and the highlight region is set, the display control unit 58 may display the highlight region for the highlight display in a color different from the original color. For example, the display control unit 58 may extract a color that is mostly included in the endoscopic image 75, and change the color of the highlight region to a color different from the colors of other portions of the endoscopic image 75. Here, the different color is, for example, a color having a different hue.

The highlight display of the highlight region is not limited thereto. The highlight display may be image processing which allows the highlight region to be visually distinguished from the surroundings, such as chroma saturation change processing, contrast processing, negative/positive inversion processing, and filtering processing. Alternatively, the highlight display of the highlight region by image processing may be combined with the highlight display by a figure surrounding the lesion portion in each of the above-described embodiments.

In each of the above-described embodiments, the four-color LEDs 20a to 20d are used to illuminate the observation target. On the other hand, a laser light source and a phosphor may be used to illuminate the observation target. In addition, in each of the above-described embodiments, the four-color LEDs 20a to 20d are used to illuminate the observation target. On the other hand, a white light light source such as a xenon lamp and a rotation filter may be used to illuminate the observation target. In addition, instead of the color imaging sensor 38, a monochrome imaging sensor may be used to perform imaging of the observation target.

In the above-described embodiments, the medical image processing apparatus according to the present invention is applied to the endoscope system that acquires an endoscopic image as a medical image. On the other hand, the medical image processing apparatus according to the present invention can be applied to various endoscope systems such as capsule endoscopes, and can also be applied to various medical imaging apparatuses that acquire, as other medical images, an X-ray image, a CT image, an MR image, an ultrasound image, a pathological image, a positron emission tomography (PET) image, and the like.

In the embodiment, a hardware structure of the processing unit that executes various processing, such as the image processing unit 56 or the display control unit 58, is realized by the following various processors. The various processors include a central processing unit (CPU) which is a general-purpose processor that functions as various processing units by executing software (program), a graphical processing unit (GPU), a programmable logic device (PLD) such as a field programmable gate array (FPGA) which is a processor capable of changing a circuit configuration after manufacture, a dedicated electric circuit which is a processor having a circuit configuration specifically designed to execute various processing, and the like.

One processing unit may be configured by one of these various processors, or may be configured by a combination of two or more processors having the same type or different types (for example, a combination of a plurality of FPGAs, a combination of a CPU and an FPGA, a combination of a CPU and a GPU, or the like). Further, the plurality of processing units may be configured by one processor. As an example in which the plurality of processing units are configured by one processor, firstly, as represented by a computer such as a client and a server, a form in which one processor is configured by a combination of one or more CPUs and software and the processor functions as the plurality of processing units is adopted. Secondly, as represented by a system on chip (SoC) or the like, a form in which a processor that realizes the function of the entire system including the plurality of processing units by one integrated circuit (IC) chip is used is adopted. As described above, the various processing units are configured by using one or more various processors as a hardware structure.

Further, as the hardware structure of the various processors, more specifically, an electric circuit (circuitry) in which circuit elements such as semiconductor elements are combined is used.

### Explanation of References

10: endoscope system
12: endoscope
12a: insertion part
12b: operating part
12c: bendable part
12d: tip part
13a: angle knob
13b: still image acquisition unit
13c: mode switching unit
13d: zoom operating part
14: light source device
16: processor device
18: monitor
19: console
20: light source unit
20a: V-LED
20b: B-LED
20c: G-LED
20d: R-LED
22: light source control unit
23: wavelength cut filter
24: light guide
30a: illumination optical system
30b: imaging optical system
32: illumination lens
34: objective lens
36: magnification optical system
36a: zoom lens
36b: lens drive unit
38: imaging sensor
40: CDS circuit
42: AGC circuit
44: A/D conversion circuit
50: image signal acquisition unit
52: DSP
54: noise reduction unit
56: image processing unit
58: display control unit
60: normal mode image processing unit
62: special mode image processing unit
64: region-of-interest detection mode image processing unit
70: detection image processing unit
71: region-of-interest detection unit
72: visibility determination unit
73: visibility notification control unit
75: endoscopic image
76: detection information
77: lesion portion
78: highlight region
79: figure
81: setting information
82: range
83: notification information
84: display screen
85A: identification information
85B: identification information
86A: icon
86B: icon
87: normal endoscopic image
88: image obtained by superimposing highlight display
89: object other than a detection target
91Ato 91D: L-shaped figures

## Claims

1. A medical image processing apparatus comprising:
a processor,
wherein the processor is configured to
acquire a medical image,
detect a region-of-interest in the medical image,
set a highlight display for highlighting the detected region-of-interest and superimpose and display the highlight display on the medical image,
determine a visibility of the highlight display from image information acquired from the medical image in which the region-of-interest is detected and the highlight display which is set, and
notify a user of a determination result of the visibility.

2. The medical image processing apparatus according to claim 1,
wherein the processor is configured to acquire the image information from an inside of the highlight display in the medical image.

3. The medical image processing apparatus according to claim 1,
wherein the processor is configured to acquire the image information from an outside of the highlight display in the medical image.

4. The medical image processing apparatus according to any one of claims 1 to 3,
wherein the processor is configured to acquire a color difference between the medical image and the highlight display from color information calculated from the image information and color information calculated from the highlight display, and determine the visibility from the color difference.

5. The medical image processing apparatus according to any one of claims 1 to 4,
wherein the processor is configured to calculate, as the color information, an average value calculated from the image information.

6. The medical image processing apparatus according to any one of claims 1 to 3,
wherein the processor is configured to display, as the highlight display, a frame-shaped figure surrounding the region-of-interest, and determine the visibility from a thickness of a line of the frame-shaped figure with respect to the region-of-interest.

7. The medical image processing apparatus according to any one of claims 1 to 3,
wherein the processor is configured to display, as the highlight display, a frame-shaped figure surrounding the region-of-interest, and determine the visibility from a similarity of the frame-shaped figure to the region-of-interest.

8. The medical image processing apparatus according to any one of claims 1 to 7,
wherein the processor is configured to display the determination result on a display screen.

9. The medical image processing apparatus according to any one of claims 1 to 8,
wherein the processor is configured to calculate a numerical index value as the determination result of the visibility, and display the determination result on a display screen.

10. The medical image processing apparatus according to claim 9,
wherein the processor is configured to display the index value as the notification in a case where the index value is equal to or smaller than a preset threshold value.

11. The medical image processing apparatus according to claim 9 or 10,
wherein the processor is configured to use, as the index value, a color difference calculated from the image information and the highlight display.

12. The medical image processing apparatus according to any one of claims 1 to 11,
wherein the processor is configured to calculate a numerical index value from the determination result of the visibility, and display identification information or an identification figure according to the index value.

13. The medical image processing apparatus according to claim 1,
wherein the processor is configured to determine the visibility based on the presence or absence of an object other than a detection target that exists in an inside of the highlight display.

14. The medical image processing apparatus according to claim 12,
wherein the processor is configured to determine that the object other than the detection target exists in a case where an area ratio of a portion at which brightness or luminance of the inside of the highlight display is equal to or higher than a second threshold value to a range in the inside of the highlight display in the medical image is equal to or higher than a third threshold value.

15. The medical image processing apparatus according to claim 8,
wherein the processor is configured to display the determination result on the display screen different from a display screen on which the medical image is displayed.

16. The medical image processing apparatus according to claim 9,
wherein the processor is configured to automatically store the medical image in which the region-of-interest is detected in a case where the index value is equal to or smaller than a preset first threshold value.

17. The medical image processing apparatus according to claim 9,
wherein the processor is configured to perform warning for a user in a case where the index value is equal to or smaller than a preset first threshold value.

18. An endoscope system comprising:
a light source device that emits an illumination light beam for illuminating an observation target;
an endoscope including an imaging sensor which images the observation target illuminated with the illumination light beam;
a processor; and
a monitor that displays a medical image obtained by performing signal processing on an image signal which is output by the imaging sensor,
wherein the processor is configured to
acquire the medical image,
detect a region-of-interest in the medical image,
set a highlight display for highlighting the detected region-of-interest and superimpose the highlight display on the medical image and display the medical image on which the highlight display is superimposed on the monitor,
determine a visibility of the highlight display from image information acquired from the medical image in which the region-of-interest is detected and the highlight display which is set, and
notify a user of a determination result of the visibility.

19. A method of operating a medical image processing apparatus, the method comprising:
a step of acquiring a medical image;
a step of detecting a region-of-interest in the acquired medical image;
a step of setting a highlight display for highlighting the detected region-of-interest and superimposing and displaying the highlight display on the medical image;
a step of determining a visibility of the highlight display from image information acquired from the medical image in which the region-of-interest is detected and the highlight display which is set; and
a step of notifying a user of a determination result of the visibility.

20. A program for a medical image processing apparatus, the program being installed in the medical image processing apparatus that acquires a medical image and performs image processing on the medical image, the program causing a computer to realize:
a function of acquiring the medical image;
a function of detecting a region-of-interest in the medical image;
a function of setting a highlight display for highlighting the detected region-of-interest and superimposing and displaying the highlight display on the medical image;
a function of determining a visibility of the highlight display from image information acquired from the medical image in which the region-of-interest is detected and the highlight display which is set; and
a function of notifying a user of a determination result of the visibility.
